# EUROPEAN PATENT SPECIFICATION

(11) **EP 3 004 746 B1**
(45) Date of publication and mention of the grant of the patent: **22.11.2017**
(21) Application number: 14725680.4
(22) Date of filing: 16.05.2014
(51) Int. Cl.: F24C 15/20, A61L 9/20, B01D 53/00

(54) **FILTER UNIT, ITS USE AND METHOD OF CLEANING CONTAMINATED AIR**
FILTEREINHEIT, DEREN VERWENDUNG UND VERFAHREN ZUM REINIGEN VON KONTAMINIERTER LUFT
UNITÉ FILTRANTE, SON UTILISATION ET PROCÉDÉ D'ÉPURATION D'AIR CONTAMINÉ

(30) Priority: 31.05.2013 SE 1350668
(43) Date of publication of application: 13.04.2016
(73) Proprietor: Acticon AB, 564 31 Bankeryd (SE)
(72) Inventor: BORANDER, Jerry, 56436 Bankeryd (SE)
(74) Representative: Awapatent AB
(86) International application number: PCT/EP2014/060036
(87) International publication number: WO 2014/191225

(56) References cited:
- WO-A1-97/07831
- DE-U1-202011 000 465
- DE-U1-202012 100 351
- GB-A- 2 487 544
- US-A1- 2004 211 321
- US-A1- 2005 000 509
- US-A1- 2008 135 041
- US-A1- 2012 152 227

## Description

### Technical field

The present disclosure relates to a filter unit, and particularly to a filter unit for use in ventilation arrangements in environments such as kitchens, where extraction of contaminated and/or hot air is desired. The present disclosure also relates to use of such filter units, and a method for filtering air in hot and or contaminated environments.

### Background

In the field of ventilation, there are a number of solutions to provide efficient extraction of air combined with efficient cleaning of the air before the air is transported back into the environment. A known solution that has proven to be efficient is to use ultraviolet (UV) light in order to treat the air to ensure that unhealthy bacteria and contaminants are destroyed before the air is returned into the environment. This solution is also used in kitchen environments, particularly in professional kitchens, where large amounts of cooking fumes and/or steam from kitchen equipment are drawn into a ventilation device and passed over a radiation source such as UV lamps in order to break down the molecules of grease and contaminants, and later transported out into ambient air. In order to provide an even more efficient cleaning of the air, the air can be treated with ozone to further break down the molecules to eliminate odours and decompose grease. The ozone is produced by the UV lamps and in cooperation with the radiation from the UV lamps, it provides an efficient cleaning in two steps, since the UV light "chops up" large molecules and the ozone then cause the remaining smaller molecules to oxidize. Since potentially inflammable grease is thus removed by the UV related treatment before the air reaches the ventilation duct, grease build up in the duct is reduced. This is advantageous, as grease build up in ventilation ducts may facilitate spreading of fire in a building, e.g. caused by a fire occurring near the ventilation unit, as may be the case in a kitchen where frying oil may accidentally catch fire or flaming may take place.

However, both UV light and ozone may be harmful to human beings as well as to organic components (e.g. components of rubber or plastic) if not handled with extreme caution, and it is of great importance that any persons around the ventilation device are shielded from the UV lamps when they are in operation. This may constitute a problem when for instance service and maintenance of the ventilation device is due, since incautious and unskilled handling may directly or indirectly harm the persons around the ventilation device. A known way to solve this is to provide the ventilation device with a pressure sensor, which automatically switches off the lamps when a service hatch is opened, and equalizes the pressure in the ventilation device with the surrounding air pressure. However, electrical components may fail due to disadvantageous environment, and hence the pressure sensor may unnoticeably stop working which may directly put any person around the ventilation device at risk when the UV lamps are exposed. Documents US 2008/0135041, US 2012/0152227 and US 2005/0000509 disclose state of the art filter units relevant to the invention.

There is therefore a need for a more safe solution to provide UV lamps in safe operation of the ventilation device independent of electrically operated safety components.

### Summary

It is an object of the present disclosure to provide a filter unit providing an improved working environment for users of the filter unit which further alleviates drawbacks of present ventilation devices.

The invention is based on the inventor's realization that due to the electrical components' sensitivity to heat and exposure to unclean air, the appropriate cooling of the electrical components and UV preventing ozone from contacting the electrical components increase the life of the arrangement and hence provide a safer working environment for those using and operating the ventilation device.

According to a first aspect, there is provided a filter unit for treating a main through flow of air. The filter unit comprises a main inlet and a main outlet in fluid connection and arranged at a distance from each other, at least one ultraviolet light source adapted for air treatment arranged between the main inlet and the main outlet, at least one electrical component for operating the ultraviolet light source. Moreover, the main flow, when the filter unit is in use, creates a negative pressure inside the filter unit between the main inlet and the main outlet. Further, the filter unit comprises a compartment separated from the main flow, in which compartment the electrical component may be arranged, wherein the compartment is provided with a secondary outlet in fluid connection with the main flow and further provided with a secondary inlet in fluid connection with an outside of the filter unit so that a secondary flow from the secondary inlet through the compartment to the secondary outlet is provided by the negative pressure inside the filter unit when in use. Further, the secondary inlet is separate from the main inlet in order to provide a secondary flow with lower temperature and/or less contaminants than in the main inlet.

In the context of this application, an inlet and an outlet "in fluid connection with each other" should be understood to mean that a flow of air or another gas is possible between the inlet and outlet. Many electrical components may be very sensitive to heat. Already at temperatures around 50-60°C, the life of electrical equipment may be significantly reduced. In a cooking environment, the air extracted into a filter unit from kitchen equipment such as grills and hobs may rise above 90°C. Insufficient cooling of the electrical equipment may thus cause them to prematurely stop working in a safe and predictable manner, or even fail.

Thus, the electrical components may be cooled using a separate flow of air, and thereby provide a safer operation of the UV lamps. The compartment may be sufficiently separate from the main flow, so that the inside of the compartment is essentially unaffected by the contaminated air in the filter unit. The compartment may be a contained space, having walls or a membrane separating the compartment from the main flow. The compartment may also be a part of the inside of the filter unit, placed close to a wall of the filter unit. However, the compartment may be formed and placed anywhere suitable in or around the filter unit. By placing the compartment inside the filter unit, the whole unit including filter unit and compartment may constitute a compact solution, allowing any equipment in connection to the filter unit be placed within one unit.

The main flow may contain hot and contaminated air to be cleaned, and may hence not be suitable for direct contact with the electrical components since the electrical components may fail due to exposure to such heat and unsanitary media. Therefore, it is an advantage to cool the electrical components by means of a secondary flow of air or any other suitable gas for cooling, which may include cleaner and/or cooler air as compared to the main flow. The secondary flow of air may be taken from a distance from the main flow and/or from a separate space, to ensure that the secondary flow, which should contain cool air in order to cool the electrical components in the compartment, is taken from a place where the air is not as hot as the air of the main flow. The secondary inlet may thus be placed sufficiently far from the main inlet to ensure that the air entering the secondary inlet is cool and clean. Alternatively the air entering the secondary inlet may be re-circulated air having been cleaned and cooled during a number of treatment steps in the filter unit, or a mixture of fresh and re-circulated air.

Some electrical components may have a function interval between for example 0-70 °C. Some electrical components may decrease its life by half for every 10 degrees increase in the environment where it may be placed. It may therefore be suitable to cool the electrical equipment so that at least the function interval may be obtained to ensure safe operation of the electrical equipment, and subsequently the operation of the filter unit and the UV lamps.

For example, the secondary flow of air may present a temperature that corresponds to an average temperature of the room in which the filter unit is placed. This temperature may be a temperature between 5 and 50 °C, more preferably between 10 and 40 °C, an even more preferably between 15 and 30 °C. Commonly, the room temperature from which the secondary flow is taken may be about 25 °C. Alternatively, the secondary air flow may be taken from another room or from outside the building.

Moreover, the secondary flow of air may present a contamination level, which is lower than that of the main flow by at least about 30 weight-%, preferably by least about 50 weight-%, or by least about 70 weight-%.

To ensure that the contamination level may be as low as possible, the secondary inlet may be placed in a space separate from the room or area that may contain any contaminants, for instance above a ceiling area. In order to further ensure that the contamination level of the air entering the compartment is very low, it is possible that a filter may be arranged upstream from the secondary inlet that may remove any contaminants that may be present.

By placing the secondary inlet of the compartment in fluid connection with an outside of the filter unit, the inlet air may be cooler and cleaner than any air inside the filter unit, which hence may be a suitable cooling agent for the electrical components. Further, the main flow and a secondary flow are fluidly connected through a secondary outlet where the secondary flow may join the main flow. The combination of having the secondary inlet on an outside of the filter unit and a secondary outlet in fluid connection with the main flow, the pressure differential between the main flow and the pressure in the compartment will cause effective suction of the secondary flow through the compartment. More particularly, the negative pressure of the main flow may cause air from the secondary inlet to be drawn through the compartment and further into the main flow, and thereby cause efficient secondary flow of cool air over the electrical components in the separate compartment.

It is advantageous if the pressure in the secondary flow is higher than in the main flow, as this would prevent ozone and contaminated air from entering into the compartment.

In the context of this application a negative pressure should be understood as a pressure lower than the pressure that may be outside of the filter unit, or the place where the secondary inlet may take its air from. The pressure of the air inside the compartment may therefore be higher than the pressure caused by the main flow.

Also, in the context of this application, by contaminated air is meant air that may need cleaning from any polluting or fouling components. Such components may be particles of ash, moisture, odours, grease or other components that may be undesirable to release into surrounding ambient air, or to introduce into a ventilation duct, after an activity using air, and thereby contaminating it.

The filter unit may be manufactured of a material sufficiently resistant to aggressive media such as grease and ozone. The material may also be resistant to the decomposing effect of ultraviolet radiation. The material may be easy to clean since it may be desirable to keep a kitchen environment hygienic for the preparation of food. For instance, the material may be a stainless steel of a grade that fulfils the relevant requirements.

The secondary inlet may be arranged at a distance from the main inlet in order to provide a secondary flow with lower temperature and/or less contaminants than in the main inlet.

Moreover, the ultraviolet light source and the compartment form part of a removable module. The removable module may be arranged so that it is possible to manually move the module. The removable module may be in the shape of a frame, having UV lamps placed suitably spaced in relation to each other so that a space between the UV lamps may contain air to be treated by the UV lamps. The space may be sufficiently dimensioned for efficient treatment of the air to occur. By allowing the module to be manually moveable, service of the lights may be facilitated. The removable module may be arranged in the filter unit, accessible from any convenient side of the filter unit. The removable module may comprise the compartment where the electrical components are placed. This would facilitate the UV lamps and its electrical components to be easily removed for cleaning or servicing.

The filter unit may be provided with at least one filter device, the filter device being received within the filter unit for, when in use, filtering the air of the main flow before the main flow reaches the ultraviolet light sources. By arranging the filter device inside the filter unit before the treatment from ultraviolet light source, large particles may be removed and the air may be cooled slightly before entering the UV lamps. The UV lamps may be sensitive to heat, and soiled air may make the UV lamps dirty which in turn affects the UV lamps ability to treat the air. A previous filtering step may thus increase the life of the UV lamps and/or the interval with which these need cleaning and make the air treatment more efficient.

The removable module may be incorporated with an openable portion of the filter unit, of which removal provides access to the interior of the filter unit. The openable portion may be arranged in a suitable place on the filter unit so that a person may access the module for cleaning or servicing of the UV lamps and/or the filter device. By incorporating the removable module in e.g. an openable hatch in the filter unit, it may provide easier access to the UV lamps, which will increase the probability of that the UV lamps being cleaned while the module is removed. Any person opening the openable portion in order to clean the filter device, may then be able to directly access the UV lamps and thereby see the need for wiping the UV lamps clean. The openable portion may thus be placed where a service hatch may be placed. The openable portion may be pivotally connected to the filter unit. The openable portion may also be fully removable or slidable in relation to the filter unit in order to expose the module.

The filter unit may comprise a safety switch arranged to disable the ultraviolet light source before access to the ultraviolet light source is enabled. There may also be a safety switch connected to an opening arrangement of the hatch, so that the hatch may not be opened unless the safety switch has been operated to disconnect any electrical connections within the module. Hence, it would be possible to ensure that any access to the module is preceded by switching off the UV lamps. This may be a safety precaution so that a person opening the hatch is not accidently exposed to harmful UV light, or any ozone produced by the UV light. The safety switch may be arranged as a keyed switch, which forces any person wanting to open the hatch to unlock the hatch and thereby disabling the electric supply. The safety switch may also be arranged as for instance pegs or micro switches in connection to the hatch. The safety switch may also be of any other convenient solution that may prevent any person from accessing the module without disabling an electrical supply to the UV lamps.

The filter unit may comprise means for preventing the removable module from being inserted into the filter unit unless the filter device is inserted in the filter unit.

The filter unit may be provided with an arrangement ensuring correct installation of the filter device and the UV lamps. Thereby it may be prevented that any part of the filter is forgotten to be mounted before the UV lamps are installed and switched on. The means for preventing incorrect installation may be any kind of obstruction that makes the removable module impossible to be installed unless the filter device is correctly installed. The obstruction may be in the form of pegs, springs, clamps or any other type of obstruction that may physically (mechanically) hinder the module from being installed unless the filter device is correctly installed in place.

The electrical components comprise at least one of the following: electrical ballast, EMC:s, cables and sockets. However, the electrical components that may be positioned in the separate compartment may be any electrical components and connections used for the operation of the UV lamps. Further, it would be possible to have other electrical component, not directly being relevant for the operation of the UV lamps, positioned in the compartment.

The filter unit may further comprise at least one pre-filtering unit arranged upstream from the main inlet. The pre-filtering unit may be arranged so that the flow of air enters the pre-filtering unit before flowing towards the filter unit and the UV lamps. The pre-filtering unit may thus add another cleaning step, where large particles and grease may be removed from the air before passing any subsequent air treating steps and over the UV lamps. The pre-filtering unit may be of any type that adds an efficient cleaning step before entering the UV lamps. Such pre-filtering unit may be of a cyclone type filter, or a labyrinth type filter which reduces the amount of particles and grease in the air.

The filter unit may be adapted to be arranged between a contamination source and a ceiling, wherein the filter unit may, but need not, be situated closer relative to said ceiling than relative to said contamination source, and wherein the secondary inlet is arranged towards the ceiling when in use. In a professional kitchen one or more filter units according to the present disclosure may be arranged. The filter unit may be arranged in a larger ventilation hood which may provide space for one or more filter units and possibly other equipment relating to the ventilation of the air. If more than one, they may be arranged together or separate between the contamination source and a ceiling, since steam and gas may be likely to rise. Depending on the size of the room, and the number of filter units to be arranged, the filter unit may be arranged close to a ceiling where connections to the air ducts may be arranged. It is also possible that the filter unit is arranged closer to the contamination source, for example in the case of intense contaminated air or that the filter unit may be of a size that will not risk obstructing the working environment.

According to a second aspect, there is also provided use of a filter unit according to any of the preceding claims for drawing hot and/or contaminated air out of a room. Although the filter unit may be used in any environment, a filter unit that may provide cooling of the electrical components in the separate compartment may be very well suited for being used in an environment where the air to be cleaned is hot and contaminated. For example, the filter unit may be installed above a cooking area, where hot and fatty fumes may arise and where grills and hobs in a kitchen may be responsible for heating the temperature of the air to around 90 degrees Celsius or more. The fat in the air may contaminate the air to an extent that it would increase the risk of a fire in the air duct due to the inflammable grease. Within a kitchen environment, the filter unit may thus be installed near a cooking station or near a dishwashing station.

The filter unit may also be suitable for use in a work shop or factory environment, where heat may arise from heavy machinery, and where the air may be contaminated due to pollutions or other contaminants. It is also possible to use a filter unit of the type in any other room where there is a need for treating air, such as a chemistry lab, office space or a home.

According to a third aspect, there is provided a method of cleaning contaminated air with a filter unit according to any of claims 1-8, comprising the steps of drawing a main flow of contaminated air through a first treatment space where an ultraviolet treatment of the contaminated air is performed and drawing a secondary flow of air of lower temperature and/or less contaminations than the main flow through the compartment separate from the main flow in which at least one electrical component (13) is arranged, such that cooling of the component may be provided, wherein the secondary flow is drawn through a secondary inlet and to the main flow, such that the step of drawing a secondary flow is achieved by means of a pressure difference between the first treatment space and the compartment, wherein said ultraviolet radiation treatment and said secondary flow through the compartment are provided in a removable module removable from the first treatment space.

### Brief Description of the Drawings

The various aspects of the invention, including its particular features and advantages, will be readily understood from the following detailed description and the accompanying drawings, in which:
Fig. 1 is a schematic view of a filter unit according to an embodiment;
Fig. 2 is a perspective view of a removable module according to an embodiment;
Fig. 3 is a schematic view of the path of the secondary flow according to an embodiment; and
Fig. 4 is an exploded perspective view of a filter unit according to an embodiment.

### Description of Embodiments

A filter unit will now be described more fully hereinafter with reference to the accompanying drawings. Like reference characters refer to like elements throughout.

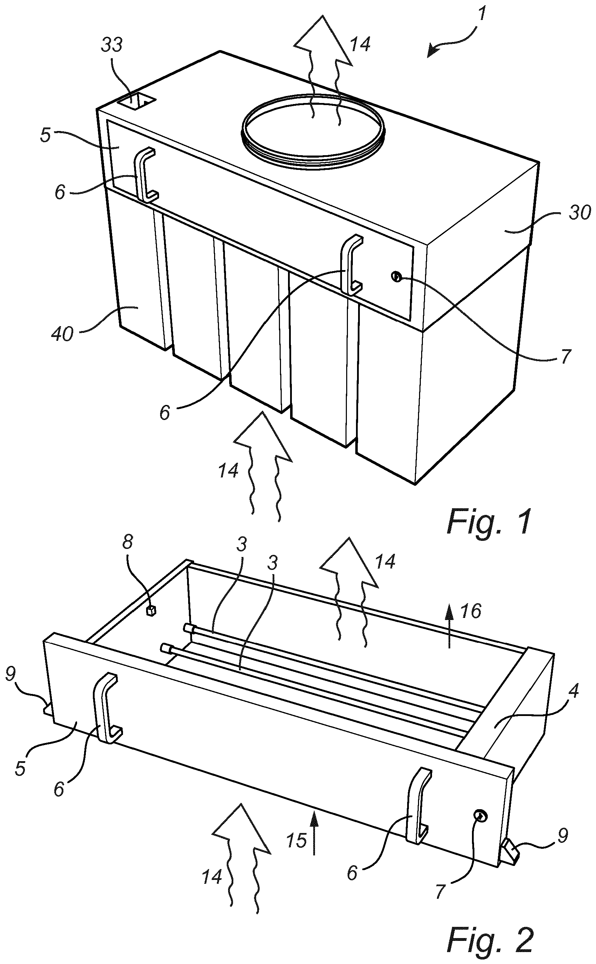

In Fig. 1 is shown a filter unit 1 according to a non-limiting embodiment of the invention, as it may appear in a kitchen environment. It is presumed when interpreting Fig. 1 that e.g. a cooking area may be placed in relation to, for instance underneath, the filter unit 1 and that e.g. the cooking fumes therefrom are extracted upwards. The filter unit is typically positioned within a ventilation hood, which operates to collect the fumes such that they may be extracted through the filter unit rather than spreading throughout the room. The ventilation hood may be mounted at a ceiling and be downwardly open while enclosing the filter unit as seen in a horizontal direction.

It is possible, but not necessary for the ventilation hood to be positioned such that it cooperates with a sub ceiling, whereby a space is formed above the filter hood and the sub ceiling and below the actual ceiling or joist structure of the room.

However, the present solution may be equally efficient if the filter unit 1 is arranged in another direction, having the air inlet placed for instance vertically relative to the cooking area, for instance as a pop-up solution in a work top or as a wall mounted arrangement or any arrangement that may provide efficient extraction of air from a room depending on the location of the contamination source. The filter unit may be arranged with regard to the size and shape of the room in which it may be arranged. It may also be arranged in a larger assembly comprising one or more filter units. The one or more filter units may be assembled within a ventilation hood.

The filter unit 1 in Fig. 1 comprises a housing 30, inside which one or more UV lamps 3 are placed. The housing 30 is provided with an openable portion in the form of a hatch 5, which allows access to the inside of the housing 30. The hatch 5 is provided with a pair of handles 6 so that easy removal of the hatch 5 is enabled.

The hatch 5 in Fig. 1 is provided with a safety switch 7 placed at the front of the filter unit 1, in the hatch 5. The safety switch 7 is present so that opening of the hatch 5 involves a step of setting the switch 7 in a safe mode, meaning that any electrical power to the UV lamps 3 is broken before opening of the hatch 5 may be allowed. The safety switch 7 may be key-operated. By turning a key in connection to the keyed switch 7, a mechanical obstacle 9 may also be released and the hatch 5 may be opened. The mechanical obstacle 9 may be for instance a sliding arrangement, a hook, latch, magnet or any other suitable arrangement that may involve mechanical hindrance if the hatch 5 is not safe to open.

By letting the operation of the keyed switch 7 involve turning of a key, unauthorised opening of the hatch 5 may be prevented since access to the key may be restricted for use only by authorised persons. The keyed switch 7 may be placed anywhere around the filter unit 1, but preferably within comfortable reach for a person wanting to open the hatch 5. The keyed switch 7 may also be replaced by some other arrangement not involving a key, for instance a turnable knob, button, wheel or any other type that may be connected to a safety switch 7 that involves breaking the electrical power before opening of the hatch 5 is possible.

One or more pre-filtering units 40 may be arranged to the filter unit 1, through which a main flow 14 of air is drawn before it enters the housing 30. The pre-filtering units 40 may be arranged to provide a first cleaning step of the air. The pre-filtering units 40 may be for instance cyclone filters or labyrinth filters, which may be arranged to provide efficient removal of larger dirt particles and grease before the air is passed to further filtering and/or air treatment steps.

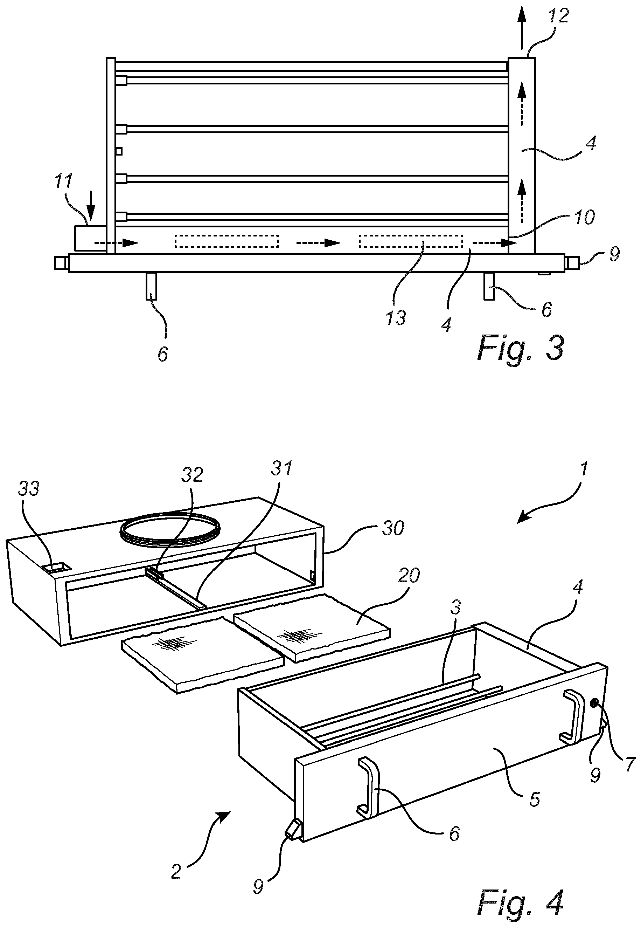

In Fig. 2 there is shown a perspective view of a removable module 2. The removable module 2 may be in the shape of a frame 2, arrangeable inside the housing 30. The module may be formed as a rectangular box, having a short height in relation to its width. The module 2 in Fig. 2 has no top or bottom walls. This is to allow a main flow 14 of air to pass through the module 2, from below towards the top. The module 2 may be provided with top and/or bottom walls, if this may be found necessary. However, it may be appropriate if such top and bottom walls are sufficiently air permeable to maintain a flow of air. Alternatively, the module may be arranged to allow the main flow 14 of air to take a different path through the module, for instance via the front, back or side walls. The module 2 may be integrated with the hatch 5, allowing the module 2 to be removed in the operation as of the removal of the hatch 5. The module 2 may comprise one or more UV light sources (UV lamps) 3 removably arranged in the module 2. The UV lamps 3 may be horizontally arranged so that the UV lamps 3 cover a significant area of which the main air flow 14 passes through the filter unit 1. The removable module 2 may comprise an enclosed compartment 4. The compartment 4 may be arranged to contain any electrical components 13, as seen in Fig. 3, used in connection to the operation of for example the UV lamps 3 and the keyed switch 7, such as EMCs, cables, connections, sockets, circuit boards, breakers and sensors. The compartment may also contain other electrical equipment 13 or equipment that may need shielding from the main flow 14 of air. As shown in Fig. 2, the compartment 4 may be enclosed to shield the contained electrical equipment 13 from any hot and contaminated air contained in the main flow 14 of air. Alternatively, the compartment 4 may be enclosed by a membrane sufficiently impermeable for the main flow 14 not to enter the compartment 4. The compartment 4 may also be placed anywhere else suitable, for instance outside the removable module 2.

The keyed switch 7 may be used for disable the UV lamps 3. The keyed switch 7 may be replaced by any other switch providing a safety function of disabling the UV lamps 3 before the removable module 2 is removed from the filter unit 1. For instance, a safety switch may be provided comprising two matching connectors (not shown) through which connectors power to the UV lamps 3 is provided. One of the connectors is arranged on the removable module 2, and the other on the housing 30 of the filter unit 1. When the removable module 2 is removed from the filter unit 1, the two connectors are separated and the UV lamps 3 are disabled. This ensures that the UV lamps 3 are disabled as soon as the removable module 2 is removed from the filter unit 1.

Fig. 3 shows a schematic view of a path of the secondary flow 10. The removable module 2 in Fig. 3 is provided with a hole that leads to an internal part of the compartment 4. This hole serves as an inlet 11 of a secondary flow 10 of air. The secondary inlet 11 may be placed in the periphery of the compartment 4, so that it is not in contact with the main air flow 14. The inlet 11 leads the secondary flow 10 of air through the inside of the compartment 4 and out through a secondary outlet 12. Due to a pressure differential between the main flow 14 which provides a negative pressure inside the filter unit 1 in the space for the treatment of the UV lamps 3, and the secondary flow 10 which is connected to ambient air and thus a higher pressure, the secondary flow 10 is by means of the negative pressure of the main flow 14 drawn through the compartment 4, across any electrical equipment 13 for cooling, and transported out through a secondary outlet 12 in fluid connection with the main flow 14 . Due to the pressure differential mentioned, any hot and contaminated air from the main flow 14 is prevented from entering the inside of the compartment 4. Hence the inside of the compartment 4 is shielded from the hot and contaminated air that may cause the electrical equipment 13 to fail. The secondary outlet 12 may be provided with a valve that prevents any air from the main flow 14 to enter, while air from the compartment 4 may enter the main flow 14, for example a one way valve, non return valve, a check valve or some other arrangement that may restrict the flow in one way.

In Fig. 4 there is shown an exploded view of a filter unit 1. The filter unit 1 comprises a housing 30, in which a removable module 2 in the shape of a frame is placeable. The removable module 2 is removable from the housing 30, and comprises at least one UV light source 3. The removable module 2 may be placed inside the housing 30 so that a compact unit may be formed.

In the bottom part of the housing 30 one or more filter devices 20 may be arranged. These filter devices 20 may be arranged to trap grease and to provide an initial cooling of the air before it reaches the module 2 with the UV lamps 3. Moreover, the filter devices 20 may reduce the amount of UV light escaping from the interior of the housing. The filter devices 20 may for example be mesh filters or the like. In a cooking environment, the air extracted into a filter unit from kitchen equipment such as grills and hobs may rise above 90 degrees Celsius.

The removable module 2 and the filter devices 20 are in one embodiment arranged such that the filter devices 20 are only removable from the filter unit 1 when the removable module 2, comprising the UV lamps 3, has been removed from the filter unit 1. Such arrangement provides that the UV lamps 3 may be cleaned at the same time as the filter devices 20 are cleaned or replaced.

The UV lamp 3 and in particular its connections are sensitive to heat so by letting the air cool before passing over the lights increases the life of the UV lamps 3, and thereby increases the reliability of the UV lamps 3 and their ability to clean contaminant air. Also, the efficiency of the UV lamps' 3 capability to break down molecules is dependent on the cleanliness of the UV lamps 3, which means that by removing a significant part of the dirty particles in the air before it reaches the UV lamps 3, may provide a better chance that the UV lamps 3 will clean the air more efficiently, since the particles to a lesser extent may stick to the UV lamps and block the air treating UV light 3.

The housing 30 is provided with a through hole 33 in the top. The hole 33 is arranged so that it is placed close to a corresponding hole in the module 2, in connection with the compartment 4, forming a secondary inlet 11 for a secondary flow 10 of air. The hole 33 should thereby preferably be placed so that fresh and cool air may enter the secondary inlet 11. The filter unit 1 may be placed so that the secondary flow 10 of air is taken relatively far from the hot and contaminated air close to the cooking area. For example, air may be taken from a space which is separated from the contaminated area by e.g. a wall, a ceiling, a joist structure or a sub ceiling.

The module 2 may be provided with handles 6 so that simple removal from the housing 30 may be possible. The handles 6 may be formed in any way and be placed anywhere so that removal of the module 2 is enabled.

The housing 30 may further be provided with a guiding arrangement 31 for receiving the filter devices 20. The guiding arrangement 31 may be arranged along a wall of the housing 30, as well as across the bottom of the housing 30. The position of the guiding arrangement 31 is dependent on the size and shape of the filter devices 20. The guiding arrangement in Fig. 4 may be provided with obstacles 32, placed in the area around where the filter devices 20 are to be placed. These obstacles 32 are provided in order to ensure that the filter devices 20 are installed correctly before insertion of the module 2 is possible. This feature is to ensure that operation of the UV lamps 3 is prevented if the filter device 20 is not present or incorrectly installed, since an incorrectly installed filter device 20 may cause the persons around the filter unit 1 to be exposed to harmful UV light and/or ozone. Possibly, the guiding arrangement 31 may be arranged directly on the removable module 2 for installation of the filter devices 20 directly in the module 2. The filter devices 20 and thus the guiding arrangement 31 may be placed anywhere suitable in the filter unit 1 depending on the need and requirements. Further, it is possible that the filter unit 1 has a shape different than rectangular. For instance, depending on the size and shape of the room, the filter unit 1 may contain passages which are not vertically arranged. The main inlet may be provided at any angle and the passages through the filter unit 1 may be drawn with regard to the requirements for that specific arrangement, for instance in a zigzag pattern, vertically or spiralled. It is also possible that the housing 30 may be interchangeable with some other arrangement. For example this may be a mounting arrangement that directly provides a slide-in option for the module, wherein the module provides the side walls.

## Claims

1. Filter unit (1) for treating a main through flow (14) of air, said filter unit (1) comprising:
a main inlet (15) and a main outlet (16) in fluid connection and arranged at a distance from each other;
at least one ultraviolet light source (3) adapted for air treatment arranged between said main inlet (15) and said main outlet (16);
at least one electrical component (13) for operating said ultraviolet light source (3);
wherein said main flow (14), when said filter unit (1) is in use, creates a negative pressure inside the filter unit (1) between said main inlet (15) and said main outlet (16); wherein said filter unit (1) further comprises a compartment (4), separated from said main flow (14), in which compartment (4) said at least one electrical component (13) is arranged, **characterized in that**
said compartment (4) is provided with a secondary outlet (12) in fluid connection with said main flow (14) and further provided with a secondary inlet (11) in fluid connection with an outside of the filter unit (1) so that a secondary flow (10) from said secondary inlet (11) through said compartment (4) to said secondary outlet (12) is provided by said negative pressure inside the filter unit (1) when in use,
wherein the secondary inlet (11) is separate from the main inlet (15) in order to provide a secondary flow (10) with lower temperature and/or less contaminants than in the main inlet, and
wherein said ultraviolet light source (3) and said compartment (4) form part of a removable module (2).

2. Filter unit (1) according to any of the preceding claims, wherein said filter unit (1) is provided with at least one filter device (20), said filter device (20) being received within said filter unit (1) for when in use, filtering the air of the main flow (14) before the main flow (14) reaches the ultraviolet light source (3).

3. Filter unit (1) according to any of claims 1-2, wherein said removable module (2) is incorporated with an openable portion (5) of the filter unit, of which removal provides access to the interior of the filter unit (1).

4. Filter unit (1) according to any of claims 1-3, wherein said filter unit comprises a safety switch (7) arranged to disable said ultraviolet light source (3) before access to said ultraviolet light source (3) is enabled.

5. Filter unit (1) according to any of the claims 1-4, wherein said filter unit (1) comprises an obstacle (32) for preventing said removable module (2) from insertion into the filter unit (1) unless said filter device (20) is inserted in the filter unit (1).

6. Filter unit (1) according to any of the preceding claims, wherein said electrical component (13) comprises at least one of an electrical ballast, an EMC, a circuit board, a cable and a lamp socket.

7. Filter unit (1) according to any of the preceding claims, wherein said filter unit (1) further comprises at least one pre-filtering device (40) arranged upstream of said main inlet (15).

8. Filter unit (1) according to any of the preceding claims, wherein said filter unit (1) is adapted to be arranged between a contamination source and a ceiling, wherein said filter unit (1) is situated closer relative to said ceiling than relative to said contamination source, and wherein said secondary inlet (11) is arranged towards the ceiling when in use.

9. Use of a filter unit (1) according to any of the preceding claims for drawing hot and/or contaminated air out of a room.

10. Method of cleaning contaminated air with a filter unit according to any of claims 1-8, comprising the steps of:
drawing a main flow (14) of said contaminated air through a first treatment space where a ultraviolet radiation treatment of said contaminated air is performed; and
drawing a secondary flow (10) of air of lower temperature and/or less contaminations than said main flow (14) through the compartment (4) which is separated from the main flow and in which at least one electrical component (13) is arranged, such that cooling of said component (13) is provided,
wherein said secondary flow (10) is drawn through a secondary inlet (11) and to said main flow (14),
such that said drawing a secondary flow (10) is achieved by means of a pressure difference between the first treatment space and the compartment (4),
wherein said ultraviolet radiation treatment and said secondary flow through the compartment are provided in a removable module (2) removable from the first treatment space.

## Patentansprüche

1. Filtereinheit (1) zum Behandeln eines Hauptdurchstroms (14) von Luft, wobei die Filtereinheit (1) Folgendes umfasst:
einen Haupteinlass (15) und einen Hauptauslass (16), die miteinander in Fluidverbindung stehen und in einem Abstand voneinander angeordnet sind;
mindestens eine Ultraviolettlichtquelle (3), die zur Luftbehandlung ausgelegt ist und zwischen dem Haupteinlass (15) und dem Hauptauslass (16) angeordnet ist;
mindestens eine elektrische Komponente (13) zum Betreiben der Ultraviolettlichtquelle (3);
wobei der Hauptstrom (14), wenn die Filtereinheit (1) in Gebrauch ist, einen Unterdruck innerhalb der Filtereinheit (1) zwischen dem Haupteinlass (15) und dem Hauptauslass (16) erzeugt;
wobei die Filtereinheit (1) ferner eine Kammer (4) umfasst, die von dem Hauptstrom (14) getrennt ist, wobei die mindestens eine elektrische Komponente (13) in der Kammer (4) angeordnet ist,
**dadurch gekennzeichnet, dass** die Kammer (4) mit einem sekundären Auslass (12) versehen ist, der mit dem Hauptstrom (14) in Fluidverbindung steht, und ferner mit einem sekundären Einlass (11) versehen ist, der mit einer Außenseite der Filtereinheit (1) in Fluidverbindung steht, so dass ein sekundärer Strom (10) von dem sekundären Einlass (11) durch die Kammer (4) zu dem sekundären Auslass (12) durch den Unterdruck innerhalb der Filtereinheit (1), wenn sie in Gebrauch ist, bereitgestellt wird,
wobei der sekundäre Einlass (11) von dem Haupteinlass (15) getrennt ist, um einen sekundären Strom (10) mit einer geringeren Temperatur und/oder weniger Verunreinigungen als der Hauptstrom bereitzustellen, und
wobei die Ultraviolettlichtquelle (3) und die Kammer (4) Teil eines entfernbaren Moduls (2) bilden.

2. Filtereinheit (1) nach einem der vorhergehenden Ansprüche, wobei die Filtereinheit mit mindestens einer Filtervorrichtung (20) ausgestattet ist, wobei die Filtervorrichtung (20) in der Filtereinheit (1) aufgenommen ist, um, wenn sie in Gebrauch ist, die Luft aus dem Hauptstrom (14) herauszufiltern, bevor der Hauptstrom (14) die Ultraviolettlichtquelle (3) erreicht.

3. Filtereinheit (1) nach einem der Ansprüche 1-2, wobei das entfernbare Modul (2) mit einem Abschnitt (5) der Filtereinheit, der geöffnet werden kann, eingebaut ist, und wobei das Entfernen desselben Zugang zum Inneren der Filtereinheit (1) bereitstellt.

4. Filtereinheit (1) nach einem der Ansprüche 1-3, wobei die Filtereinheit einen Sicherheitsschalter (7) umfasst, der dazu ausgelegt ist, die Ultraviolettlichtquelle (3) zu deaktivieren, bevor Zugang zu der Ultraviolettlichtquelle (3) ermöglicht wird.

5. Filtereinheit (1) nach einem der Ansprüche 1-4, wobei die Filtereinheit (1) ein Hindernis (32) umfasst, um zu verhindern, dass das entfernbare Modul (2) in die Filtereinheit (1) eingesetzt wird, wenn die Filtervorrichtung (20) nicht in die Filtereinheit (1) eingesetzt ist.

6. Filtereinheit (1) nach einem der vorhergehenden Ansprüche, wobei die elektrische Komponente (13) ein Vorschaltgerät und/oder EMV und/oder eine Platine und/oder ein Kabel und/oder eine Fassung umfasst.

7. Filtereinheit (1) nach einem der vorhergehenden Ansprüche, wobei die Filtereinheit (1) ferner mindestens eine Vorfilterungsvorrichtung (40) umfasst, die stromaufwärts des Haupteinlasses (15) angeordnet ist.

8. Filtereinheit (1) nach einem der vorhergehenden Ansprüche, wobei die Filtereinheit (1) dazu ausgelegt ist, zwischen einer Verunreinigungsquelle und einer Decke angeordnet zu sein, wobei die Filtereinheit (1) näher zur Decke als zur Verunreinigungsquelle angeordnet ist und wobei der sekundäre Einlass (11) in Gebrauch in Richtung der Decke angeordnet ist.

9. Verwendung einer Filtereinheit (1) nach einem der vorhergehenden Ansprüche, zum Abziehen von heißer und/oder verunreinigter Luft aus einem Zimmer.

10. Verfahren zum Reinigen von verunreinigter Luft mit einer Filtereinheit nach einem der Ansprüche 1-8, das die folgenden Schritte umfasst:
Abziehen eines Hauptstroms (14) der verunreinigten Luft durch einen ersten Behandlungsraum, wo eine Ultraviolettbestrahlungsbehandlung der verunreinigten Luft durchgeführt wird; und
Abziehen eines sekundären Stroms (10) von Luft mit einer geringeren Temperatur und/oder weniger Verunreinigungen als der Hauptstrom (14) durch die Kammer (4), die von dem Hauptstrom getrennt ist und in der die mindestens eine elektrische Komponente (13) angeordnet ist, derart, dass ein Abkühlen der Komponente (13) bereitgestellt wird,
wobei der sekundäre Strom (10) durch einen sekundären Einlass (11) und zu dem Hauptstrom (14) abgezogen wird, derart dass das Abziehen eines sekundären Stroms (10) mittels eines Druckunterschieds zwischen dem ersten Behandlungsraum und der Kammer (4) erreicht wird,
wobei die Ultraviolettbestrahlungsbehandlung und der sekundäre Strom durch die Kammer in einem entfernbaren Modul (2) bereitgestellt werden, das von dem ersten Behandlungsraum entfernbar ist.

## Revendications

1. Unité filtrante (1) pour le traitement d'un écoulement principal (14) d'air, ladite unité filtrante (1) comprenant :
une entrée principale (15) et une sortie principale (16) en connexion fluidique et agencées à une distance l'une de l'autre ;
au moins une source lumineuse d'ultraviolets (3) adaptée pour le traitement de l'air agencée entre ladite entrée principale (15) et ladite sortie principale (16) ;
au moins un composant électrique (13) pour faire fonctionner ladite source lumineuse d'ultraviolets (3) ;
dans laquelle ledit écoulement principal (14), lorsque ladite unité filtrante (1) est en utilisation, crée une pression négative à l'intérieur de l'unité filtrante (1) entre ladite entrée principale (15) et ladite sortie principale (16) ;
dans laquelle
ladite unité filtrante (1) comprend en outre un compartiment (4) séparé dudit écoulement principal (14), dans lequel compartiment (4) est agencé ledit au moins un composant électrique (13), **caractérisé en ce que**
ledit compartiment (4) est doté d'une sortie secondaire (12) en connexion fluidique avec ledit écoulement principal (14) et en outre doté d'une entrée secondaire (11) en connexion fluidique avec un extérieur de l'unité filtrante (1) de façon à ce qu'un écoulement secondaire (10) venant de ladite entrée secondaire (11) à travers ledit compartiment (4) vers ladite sortie secondaire (12) soit fourni par ladite pression négative à l'intérieur de l'unité filtrante (1) en cours d'utilisation,
dans laquelle l'entrée secondaire (11) est séparée de l'entrée principale (15) afin de fournir un écoulement secondaire (10) avec une température inférieure et/ou moins de contaminants que dans l'entrée principale, et
dans laquelle ladite source lumineuse d'ultraviolets (3) et ledit compartiment (4) font partie d'un module amovible (2).

2. Unité filtrante (1) selon l'une quelconque des revendications précédentes, dans laquelle ladite unité filtrante (1) est dotée d'au moins un dispositif filtrant (20), ledit dispositif filtrant (20) étant reçu à l'intérieur de ladite unité filtrante (1) pour, lors de l'utilisation, filtrer l'air de l'écoulement principal (14) avant que l'écoulement principal (14) n'atteigne la source lumineuse d'ultraviolets (3).

3. Unité filtrante (1) selon l'une quelconque des revendications 1-2, dans laquelle ledit module amovible (2) est incorporé avec une partie ouvrable (5) de l'unité filtrante, dont le retrait fournit l'accès à l'intérieur de l'unité filtrante (1).

4. Unité filtrante (1) selon l'une quelconque des revendications 1-3, dans laquelle l'unité filtrante comprend un interrupteur de sécurité (7) agencé pour désactiver ladite source lumineuse d'ultraviolets (3) avant que l'accès à ladite source lumineuse d'ultraviolets (3) ne soit activé.

5. Unité filtrante (1) selon l'une quelconque des revendications 1-4, dans laquelle l'unité filtrante (1) comprend un obstacle (32) pour éviter que ledit module amovible (2) ne soit inséré dans l'unité filtrante (1) à moins que ledit dispositif filtrant (20) ne soit inséré dans l'unité filtrante (1).

6. Unité filtrante (1) selon l'une quelconque des revendications précédentes, dans laquelle ledit composant électrique (13) comprend au moins un d'un ballast électrique, d'un EMC, d'une carte de circuit, d'un câble et d'une douille de lampe.

7. Unité filtrante (1) selon l'une quelconque des revendications précédentes, dans laquelle ladite unité filtrante (1) comprend en outre au moins un dispositif de pré-filtrage (40) agencé en amont de ladite entrée principale (15).

8. Unité filtrante (1) selon l'une quelconque des revendications précédentes, dans laquelle ladite unité filtrante (1) est adaptée pour être agencée entre une source de contamination et un plafond, dans laquelle ladite unité filtrante (1) est située plus près par rapport audit plafond que par rapport à ladite source de contamination, et dans lequel ladite entrée secondaire (11) est agencée en direction du plafond en utilisation.

9. Utilisation d'une unité filtrante (1) selon l'une quelconque des revendications précédentes pour entraîner de l'air chaud et/ou contaminé hors d'une pièce.

10. Procédé d'épuration d'air contaminé comprenant une unité filtrante selon l'une quelconque des revendications 1 - 8, comprenant les étapes :
d'entraînement d'un écoulement principal (14) dudit air contaminé à travers un premier espace de traitement où un traitement aux rayons ultraviolets dudit air contaminé est effectué ; et
entraîner un écoulement secondaire (10) d'air avec une température inférieure et/ou moins de contaminants que dans ledit écoulement principal (14) à travers le compartiment (4) qui est séparé de l'écoulement principal et dans lequel au moins un composant électrique (13) est agencé de telle façon que le refroidissement dudit composant (13) est fourni,
dans lequel ledit écoulement secondaire (10) est entraîné à travers une entrée secondaire (11) et vers ledit écoulement principal (14), de telle façon que ledit entraînement d'un écoulement secondaire (10) est obtenu au moyen d'une différence de pression entre le premier espace de traitement et le compartiment (4),
dans lequel ledit traitement aux rayons ultraviolets et ledit écoulement secondaire à travers le compartiment sont fournis dans un module amovible (2) pouvant être enlevé du premier espace de traitement.
